# EUROPEAN PATENT SPECIFICATION

(11) **EP 2 974 802 B1**
(45) Date of publication and mention of the grant of the patent: **29.04.2020**
(21) Application number: 15152721.5
(22) Date of filing: 27.01.2015
(51) Int. Cl.: B06B 1/06, G10K 11/00

(54) **ULTRASOUND PROBE INCLUDING A BACKING MEMBER**
ULTRASCHALLSONDE MIT EINEM TRÄGERKÖRPER
SONDE À ULTRASONS COMPRENANT UN ÉLÉMENT DE SUPPORT

(30) Priority: 14.07.2014 KR 20140088449
(43) Date of publication of application: 20.01.2016
(73) Proprietor: Samsung Medison Co., Ltd., Hongcheon-gun, Gangwon-do (KR); Samsung Electronics Co., Ltd., Gyeonggi-do 443-742 (KR)
(72) Inventor: LEE, Won-hee, Hongcheon-gun, Gangwon-do (KR); SON, Kwang-jin, Hongcheon-gun, Gangwon-do (KR); SONG, Kyung-hun, Hongcheon-gun, Gangwon-do (KR); KIM, Sang-min, Gangdong-gu, Seoul (KR); NAM, Gi-ung, Hongcheon-gun, Gangwon-do (KR); LEE, Sung-jae, Hongcheon-gun, Gangwon-do (KR)
(74) Representative: Grootscholten, Johannes A.M.

(56) References cited:
- EP-A2- 2 842 642
- WO-A2-2013/140283
- US-A- 3 376 438
- US-A1- 2005 127 793
- US-A1- 2012 238 880
- US-A1- 2013 172 750
- US-B2- 7 046 583

## Description

### BACKGROUND

### 1. Field

One or more embodiments of the present invention relate to an ultrasound backing member and an ultrasound probe including the ultrasound backing member.

### 2. Description of the Related Art

Ultrasound diagnosis apparatuses display images in real time, are small and inexpensive, and are safe due to minimal exposure to radiation, compared to other image diagnosis apparatuses, such as, X-ray apparatuses, computerized tomography (CT) scanners, magnetic resonance imaging (MRI) apparatuses, and nuclear medical diagnosis apparatuses. Therefore, ultrasound diagnosis apparatuses are widely used for diagnoses of the heart, the abdomen, the unitary system, and obstetrics and gynecology.

Ultrasound diagnosis apparatuses include an ultrasonic probe that transmits an ultrasound signal to an object and receives an echo signal reflected from the object to obtain an image of the object.

The ultrasonic probe may generate heat while operating. In particular, a transducer that converts ultrasound waves into an electrical signal or vice versa may generate a lot of heat. To maintain the performance of ultrasonic probes, heat needs to be efficiently discharged.

Here, US-2012/238880 is acknowledged as prior art in relation to the present disclosure, relative to which at least features in the characterizing portions of the appended independent claims are novel and involve an inventive step. EP-2842642 constitutes prior art under art. 54(3) EPC, and US-2013/172750 and US-2005/127793 are acknowledged as secondary prior art.

### SUMMARY

One or more embodiments of the present invention include an ultrasound backing member having improved heat conduction, and an ultrasound probe including the ultrasound backing member.

Additional aspects will be set forth in part in the description which follows and, in part, will be apparent from the description, or may be learned by practice of the presented embodiments.

According to one or more embodiments of the present invention, an ultrasound probe comprises all aspects and features of the appended independent probe claim 1. Preferred embodiments may be defined in dependent claims.

### BRIEF DESCRIPTION OF THE DRAWINGS

These and/or other aspects will become apparent and more readily appreciated from the following description of the embodiments, taken in conjunction with the accompanying drawings in which:
FIG. 1 is a block diagram of an ultrasound diagnosis apparatus according to an embodiment of the present invention;
FIG. 2 is a block diagram of an ultrasonic module included in the ultrasound diagnosis apparatus illustrated in FIG. 1;
FIG. 3 is a schematic view of an ultrasound probe according to an embodiment;
FIG. 4 illustrates a porous foam-type body according to an embodiment;
FIG. 5 illustrates a backing member obtained by filling the foam-type body of FIG. 4 with a backing material, according to an embodiment;
FIG. 6 illustrates a heat transfer path in the backing member of FIG. 5; and
FIG. 7 is a flowchart of a method of manufacturing a backing member, according to an example not forming part of the invention.

### DETAILED DESCRIPTION

Embodiments of the invention now will be described more fully hereinafter with reference to the accompanying drawings, in which exemplary embodiments of the invention are shown. In the drawings, like elements are denoted by like reference numerals, and a repeated explanation thereof will not be given.

The term "object" used herein may be a human, an animal, or a body part of a human or animal. For example, the object may be an organ (e.g., the liver, the heart, the womb, the brain, a breast, or the abdomen), a blood vessel, or a combination thereof. The term "user" may be, but is not limited thereto, a medical expert, such as a medical doctor, a nurse, a health care technician, or a medical imaging expert, or may be an engineer who repairs medical apparatuses.

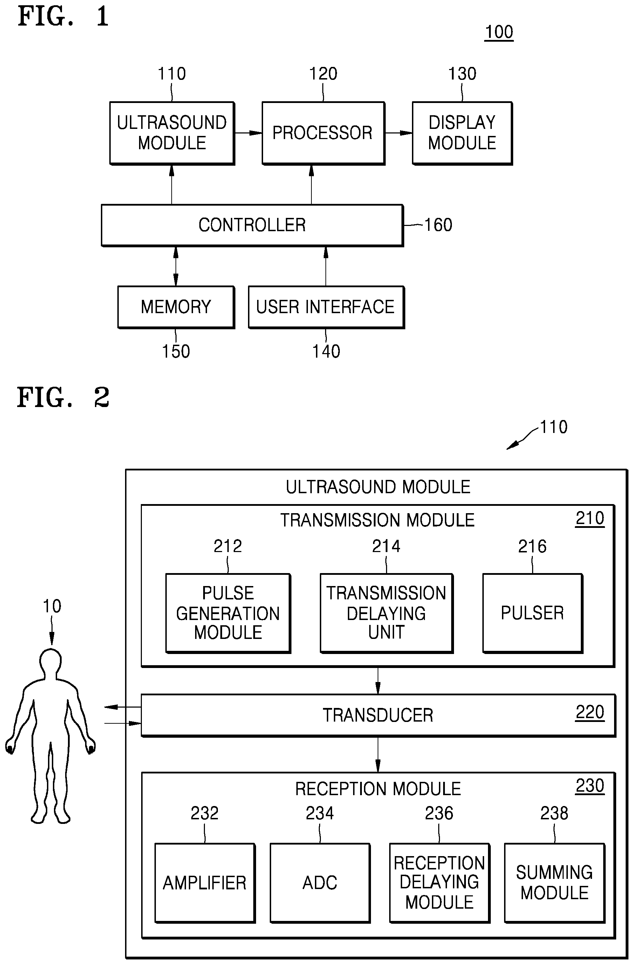

FIG. 1 is a block diagram of an ultrasound diagnosis apparatus 100 according to an embodiment of the present invention. Referring to FIG. 1, the ultrasound diagnosis apparatus 100 includes an ultrasound module 110 that transmits or receives ultrasonic waves, a processor 120 that processes a signal received from the ultrasound probe 110 to generate an image, a display module 130 that displays the image, a user interface 140 that receives an input of a user command, a memory 150 that stores various pieces of information, and a controller 160 that controls overall operations of the ultrasound diagnosis apparatus 100.

The ultrasound module 110 transmits ultrasound waves to an object and receives an echo signal corresponding to ultrasound waves reflected from the object, as will be described later in detail.

The processor 120 generates an ultrasound image by processing ultrasound data generated by the ultrasound module 110. The ultrasonic image may be at least one of a brightness (B) mode image representing a magnitude of an ultrasonic echo signal by brightness, a Doppler mode image representing an image of a moving object in the form of a spectrum by using the Doppler effect, a motion (M) mode image representing a time-dependent motion of an object at a predetermined position, an elasticity mode image representing a difference between a reaction of an object when compression is applied to the object and a reaction of the object when compression is not applied to the object by an image, and a color (C) mode image representing a speed of a moving object by color by using the Doppler effect. The ultrasound image may be generated using a currently-usable ultrasound image generating method, and thus, a detailed description thereof will be omitted herein. Accordingly, in the present embodiment, the ultrasound image may be any of images obtained in dimensional modes, such as a one-dimensional (1D) mode, a two-dimensional (2D) mode, a three-dimensional (3D) mode, and a four-dimensional (4D) mode.

The display module 130 displays information processed by the ultrasound diagnosis apparatus 100. For example, the display module 130 may display the ultrasound image generated by the processor 120, and may also display a graphical user interface (GUI) or the like for requesting a user input.

The display module 130 may be at least one selected from a liquid crystal display (LCD), a thin film transistor-liquid crystal display (TFT-LCD), an organic light-emitting diode (OLED), a flexible display, a 3D display, and an electrophoretic display. In some cases, the ultrasound diagnosis apparatus 100 may include at least two display modules 130.

The user interface 140 refers to a unit via which a user inputs data for controlling the ultrasound diagnosis apparatus 100. The user interface 140 may include a key pad, a mouse, a touch pad, a trackball, and the like. The user interface 140 is not limited thereto, and thus may further include various input units such as a jog wheel and a jog switch.

A touch pad may detect not only a real touch, but may also detect a proximity touch. The real touch means a case in which a pointer actually touches a screen, and the proximity touch means a case in which the pointer approaches the screen but does not actually touch the screen. In this specification, the pointer is a tool used for actually touching or proximately touching a predetermined portion of the touch pad. Examples of the tool include a stylus pen, a part of a body such as a finger, and the like.

The touch pad may be implemented as a touch screen that forms a multiple-layer structure together with the display module 130. The touch screen may be variously embodied as a capacitive type touch screen, a pressure resistive type touch screen, an infrared beam sensing type touch screen, a surface acoustic wave type touch screen, an integral strain gauge type touch screen, a piezoelectric effect type touch screen, or the like. Since a touch screen performs the functions of both the display module 130 and the user interface 140, the touch screen is highly utilized.

Although not illustrated in FIG. 1, in order to detect a touch, a touch pad may include various sensors provided within or near the touch pad. An example of the sensor used to detect a touch on the touch pad is a tactile sensor. The tactile sensor detects a contact of a specific object with a sensitivity that is equal to or greater than human perception. The tactile sensor may detect various types of information, such as the roughness of a touched surface, the hardness of the touching object, and the temperature of a touched point.

Another example of the sensor used to detect a touch on the touch pad is a proximity sensor. The proximity sensor detects the existence of an object that approaches a predetermined detection surface or that exists nearby, by using an electromagnetic force or infrared rays without any mechanical contact. Examples of the proximity sensor include a transmission-type photoelectric sensor, a direct reflection-type photoelectric sensor, a mirror reflection-type photoelectric sensor, a high frequency oscillation-type proximity sensor, a capacity-type proximity sensor, a magnetic proximity sensor, and an infrared-type proximity sensor.

The memory 150 stores various pieces of information processed by the ultrasound diagnosis apparatus 100. For example, the memory 150 may store medical data related to diagnosis of an object, such as an image, and may also store algorithms or programs which are to be executed in the ultrasound diagnosis apparatus 100.

The memory 150 may include at least one type of storage medium selected from among a flash memory type, a hard disk type, a multimedia card micro type, a card type memory (for example, a secure digital (SD) or extreme digital (XD) memory), a random access memory (RAM), a static random access memory (SRAM), a read-only memory (ROM), an electrically erasable programmable ROM (EEPROM), a programmable ROM (PROM), magnetic memory, a magnetic disk, and an optical disk. The ultrasound diagnosis apparatus 100 may utilize web storage or a cloud server which performs the storage function of the memory 150, but online.

The controller 160 controls all operations of the ultrasound diagnosis apparatus 100. That is, the controller 160 may control operations performed by the ultrasonic module 110, the processor 120, the display module 130, and the like shown in FIG. 1. For example, the controller 160 may control the processor 120 to produce an image by using a user command that is input via the user interface 140 or by using a program stored in the memory 150. The controller 160 may also control the display module 130 to display the image produced by the processor 120.

FIG. 2 is a block diagram of the ultrasonic module 110 illustrated in FIG. 1. Referring to FIG. 2, the ultrasound module 110 may transmit ultrasound waves to an object 10 and receive an echo signal reflected from the object 10 to generate ultrasound data. The ultrasound module 110 may include a transmission module 210, a transducer 220, and a reception module 230.

The transmission module 210 provides a driving signal to the transducer 220. The transmission module 210 may include a pulse generation module 212, a transmission delaying unit 214, and a pulser 216.

The pulse generation module 212 generates rate pulses for generating transmission ultrasound waves based on a predetermined pulse repetition frequency (PRF). The transmission delaying module 214 delays the rate pulses generated by the pulse generation module 212 by delay times for determining transmission directionality. Each of the rate pulses which have been delayed correspond to a plurality of unit elements included in the transducer 220, respectively. The pulser 216 applies a driving signal or a driving pulse to the transducer 220 based on timing that corresponds to each of the rate pulses which have been delayed. The plurality of unit elements may be arranged in a 1D array or a 2D array.

The transducer 220 transmits ultrasound waves to the object 10 in response to the driving signal applied by the transmission module 210 and receives an echo signal corresponding to ultrasound waves reflected by the object 10. The transducer 220 may include a plurality of unit elements that convert electrical signals into acoustic energy or vice versa. The plurality of unit elements may be arranged in a 1D array or a 2D array.

The transducer 220 may be a piezoelectric micromachined ultrasonic transducer (pMUT) that converts ultrasound waves into an electrical signal or vice versa according to a pressure change by vibrating, a capacitive micromachined ultrasonic transducer (cMUT) that converts ultrasound waves into an electrical signal or vice versa due to a change in an electrostatic capacity, a magnetic micromachined ultrasonic transducer (mMUT) that converts ultrasound waves into an electrical signal or vice versa based on a change in a magnetic field, or an optical ultrasonic detector that converts ultrasound waves into an electrical signal or vice versa according to a change in optical characteristics.

The reception module 230 may generate the ultrasound data by processing a signal received from the transducer 220. The reception module 230 may include an amplifier 232, an analogue-to-digital converter (ADC) 234, a reception delaying module 236, and a summing module 238.

The amplifier 232 amplifies the signal received from the transducer 220, and the ADC 234 performs analogue-to-digital conversion on the amplified signal. The reception delaying unit 236 delays a digital signal output by the ADC 234 by a delay time necessary for determining reception directionality. The summing module 238 generates the ultrasound data by summing signals processed by the reception delaying module 236. A reflection component in a direction determined by the reception directionality may be emphasized by a summing operation of the summing module 238.

The ultrasound module 110 may necessarily include the transducer 220, but at least some of the transmission module 210 and the reception module 230 may be included in another apparatus. For example, the ultrasound module 110 may not include the summing module 238 of the reception module 230.

The transmission module 210 and the reception module 230 of the ultrasound module 110 may be formed on a single substrate in the form of at least one chip. Here, the single substrate may be formed of silicon (Si), ceramic, or a polymer-based material. Each block included in the transmission module 210 and the reception module 230 may be formed as a single chip, at least two blocks may be formed as a single chip, or respective blocks of the transmission module 210 and the reception module 230 corresponding to a unit element of the transducer 220 may form a single chip together with the unit element of the transducer 220. Thus, a substrate including at least one of the transmission module 210 and the reception module 230 is referred to as a chip module substrate. The chip module substrate may denote a substrate including all of the chips included in the ultrasound module 110 or may also denote a substrate including some of the chips included in the ultrasound module 110.

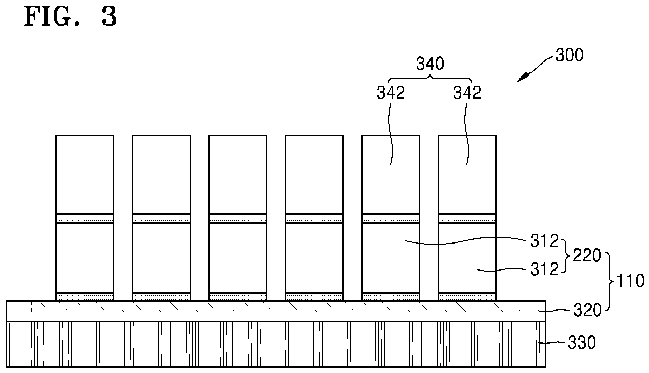

A physical structure including the ultrasound module 110 is referred to as an ultrasound probe 300. FIG. 3 is a schematic view of the ultrasound probe 300 according to an embodiment. As illustrated in FIG. 3, the ultrasound probe 300 includes the ultrasound module 110 which converts an electrical signal into ultrasound waves or vice versa, a backing member 330 which absorbs ultrasound waves that are transmitted in a direction going away from the object, and a matching member 340 which matches an acoustic impedance of the ultrasound waves generated by the ultrasound module 110 with an acoustic impedance of an object.

The ultrasound module 110 may include the transducer 220 and a chip module substrate 320. FIG. 3 illustrates piezoelectric elements 312 as the unit elements of the transducer 220. While vibrating, the piezoelectric elements 312 may convert an electrical signal into an acoustic signal or vice versa. The piezoelectric elements 312 may be formed by splitting a piezoelectric material. For example, the piezoelectric elements 312 may be manufactured by dicing a piezoelectric material that extends lengthwise. However, a method of manufacturing the piezoelectric elements 312 is not limited to this dicing method, and the piezoelectric elements 312 may be manufactured using various other methods, such as, a method of pressing a piezoelectric material by using a metal mold. The piezoelectric material may include at least one selected from ZnO, AlN, PZT(PbZrTiO3), PLZT(PbLaZrTiO3), BT(BaTiO3), PT(PbTiO3), PMN-PT(Pb(Mg1/3Nb2/3)O3-PbTiO3), and the like that cause a piezo phenomenon.

Although the piezoelectric elements 312 are illustrated as the unit elements of the transducer 220 in FIG. 3, this is only an example. The transducer 220 may include capacitive elements that convert ultrasound waves into an electrical signal or vice versa due to a change in an electrostatic capacity, magnetic elements that convert ultrasound waves into an electrical signal or vice versa due to a change in a magnetic field, or optical elements that convert ultrasound waves into an electrical signal or vice versa according to a change in optical characteristics.

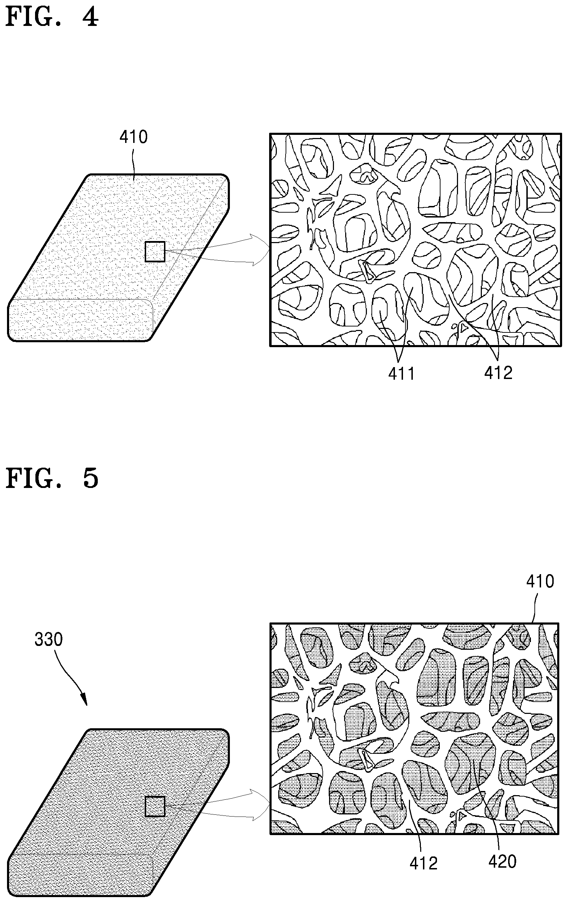

The backing member 330 may absorb ultrasound waves that are transmitted in a direction going away from the object. The backing member 330 may support the chip module substrate 320 at the back surface of the chip module substrate 320 and absorb ultrasound waves that are transmitted toward the back surface of the transducer 220 and are thus not directly used in tests or diagnosis. Although the backing member 330 is separate from the chip module substrate 320, this is only for convenience of explanation, and thus embodiments of the present invention are not limited thereto. The chip module substrate 320 may be formed of a backing material 420 (see FIG. 5). Thus, the chip module substrate 320 may perform the function of the backing member 330.

The ultrasound probe 300 may also include the matching member 340 which matches the acoustic impedance of the ultrasound waves generated by the transducer 220 with the acoustic impedance of the object. The matching member 340 is disposed on a front surface of the transducer 220 and changes the acoustic impedance of the ultrasound waves generated by the transducer 220 in stages so that the acoustic impedance of the ultrasound waves is approximate to the acoustic impedance of the object. The front surface of the transducer 220 may be a surface that is closest to the object from among the surfaces of the transducer 220 when ultrasound waves are applied to the object, and a rear surface thereof may be a surface opposite to the front surface.

The matching member 340 may be formed of a plurality of matching elements 342 respectively disposed on the piezoelectric elements 312. However, the present embodiment is not limited thereto. The matching member 340 may be a single matching element into which the plurality of piezoelectric elements 312 are grouped. The matching member 340 may have a single-layered structure or a multi-layered structure.

The ultrasound probe 300 may further include an acoustic lens (not shown) that focuses ultrasound waves. The acoustic lens is disposed on the front surface of the transducer 220 and focuses the ultrasound waves generated by the transducer 220. The acoustic lens may be formed of a material such as silicon rubber having an acoustic impedance that is similar to that of the object. A central portion of the acoustic lens may be convex or flat. The acoustic lens may have various shapes according to designs of manufacturers.

While the ultrasound probe 300 is generating and collecting ultrasound waves, the piezoelectric elements 312 included in the ultrasound module 110 inevitably vibrate, and heat is generated due to this vibration. In addition, since components included in ultrasound probes are highly integrated with miniaturization of the ultrasound probes, the amount of generated heat increases in embodiments of the present invention compared with in the conventional art.

If the heat generated in this way fails to be discharged to the outside and is transmitted to the acoustic lens of the ultrasound probe 300, the heat may be transferred directly to the skin of a patient. Thus, the number of times a patient gets burned may increase. The functions of components included in the ultrasound probe 300 may be degraded due to a lot of heat, and accordingly negative effects may be exerted on the safety of a patient and a diagnostic image. Therefore, the ultrasound probe 300 needs to effectively discharge generated heat.

A backing member included in an ultrasound probe according to an embodiment includes a porous foam-type body and a backing material that fills the foam-type body and absorbs ultrasound waves. FIG. 4 illustrates a porous foam-type body 410 according to an embodiment. FIG. 5 illustrates a backing member 330 obtained by filling the foam-type body 410 of FIG. 4 with the backing material 420, according to an embodiment. As illustrated in FIG. 4, the foam-type body 410 includes a plurality of pores 411. In detail, the foam-type body 410 includes the pores 411 and a plurality of bridges 412 connected to one another and located between the pores 411. The bridges 412 are connected to one another and include metal. The metal is a highly heat-conductive material, such as copper, aluminum, silver, nickel, iron, gold, or tungsten. The bridges 412 of the foam-type body 410 are connected to one another without disconnections to serve as a passage via which heat generated in an ultrasound probe is discharged to the outside. Thus, the heat generated in the ultrasound module 110 is externally discharged via the foam-type body 410, for example, the bridges 412.

The foam-type body 410 includes the pores 411. At least some of the pores 411 may overlap with each other. In other words, a pore 411 may be connected to at least one of neighboring pores 411. Accordingly, an external material may flow into the foam-type body 410 via the pores 411. Sizes of the pores 411 do not need to be constant, and thus may be irregular. A porosity of the foam-type body 410 may be about 50% or more. A diameter of each pore 411 may be, but is not limited to, about 1 mm to about 10 mm. The diameter of each of the pores 411 may vary according to methods of manufacturing the foam-type body 410.

The foam-type body 410 may be manufactured using a bottom-up method, a method using a freeze-drying solution, or a method using a foam base. However, a method of manufacturing the foam-type body 410 is not limited thereto.

When the backing member 330 includes only the foam-type body 410, the backing member 330 may not properly absorb ultrasound waves. When ultrasound waves are not absorbed but are transmitted or reflected by the backing member 330, a distorted ultrasound image may be obtained. In the backing member 330, the pores 411 may be filled with the backing material 420. The backing material 420 may include a material having a high attenuation coefficient. For example, the backing material 420 may include epoxy resin. Alternatively, the backing material 420 may be a combination of a metal powder (for example, tungsten, copper, or aluminum), a ceramic powder (for example, tungsten oxide or alumina), or a carbon allotrope powder (for example, graphite, graphene, carbon nanotubes, or diamond) with epoxy resin, or may include rubber.

As described above, since the foam-type body 410 may include the plurality of pores 411, the pores 411 may be filled with the backing material 420. In addition, since heat generated in the transducer 220 is discharged to the outside via the foam-type body 410, heat transferred to a patient via a lens may be effectively reduced. When a heat discharge is effectively performed, the safety of patients may be secured, and a degradation of the function of an ultrasound diagnosis apparatus due to a lot of heat may be prevented. Moreover, when power is applied to generate ultrasound waves, higher power may be applied in consideration of the degree of heat discharge, and thus a clear ultrasound image may be obtained.

Since the ultrasound probe 300 includes the backing member 330, the ultrasound probe 300 may perform a heat discharge function while keeping a conventional shape and size, without including a special heat-discharging apparatus.

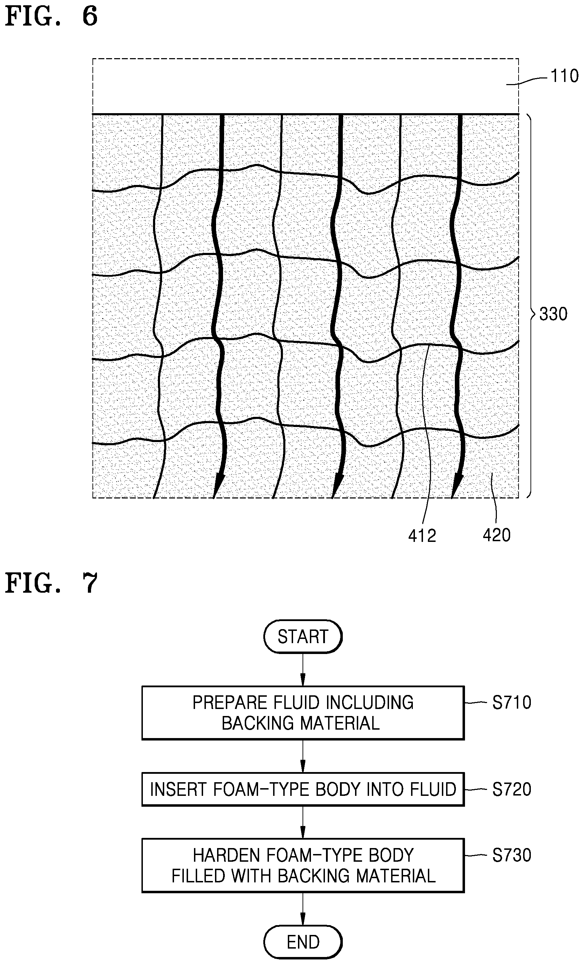

FIG. 6 illustrates a heat transfer path in the backing member 330 of FIG. 5. In FIG. 6, arrows indicate paths in which heat generated in the ultrasound module 110 is discharged via the backing member 330. Most of the heat generated in the ultrasound module 110 is generated in the transducer 220, because the transducer 220 vibrates in response to a voltage and generates an electrical signal by vibrating in response to echo ultrasound waves. The backing member 330 may discharge the heat generated in the ultrasound module 110 to the outside. In detail, since the foam-type body 410 of the backing member 330 includes the bridges 412 formed of a highly heat-conductive material, heat may be discharged to the outside via the bridges 412 at high speed.

Although not shown in FIG. 3, a heat sink may be provided on a lower surface of the backing member 330. Since the heat sink may effectively discharge heat, the heat sink may thermally and directly contact the backing member 330 to help a heat discharge. Alternatively, instead that the heat sink directly contacts the backing member 330, the backing member 330 and the heat sink may be connected to each other via a heat pipe. In this case, heat travelling via the backing member 330 is transferred to the heat sink along the heat pipe and is then discharged to the outside.

According to an example not forming part of the invention, a heat discharging fan may also be provided to help the heat sink to discharge heat. The heat sink may include a plurality of fins that each have a plate shape and are formed of a metal, such as aluminum, to disperse heat. Heat transferred to the heat sink is dispersed by the plurality of fins, and the heat discharging fan may be disposed near the fins to additionally improve the heat discharge function.

The backing member 330 is grounded. Due to the grounding of the backing member 330, interference among electrical signals within the ultrasound probe 300 is prevented. Moreover, the backing member 330 may serve as a unit that applies a reference voltage, for example, a ground signal, to the transducer 220. In this case, the backing member 330 may contact the transducer 220.

FIG. 7 is a flowchart of a method of manufacturing the backing member 330, according to an example not forming part of the invention.

First, in operation S710, a fluid including the backing material 420 absorbing ultrasound waves is prepared. The backing material 420 may include a material having a high attenuation coefficient. For example, the backing material 420 may include epoxy resin. Alternatively, the backing material 420 may be a combination of a metal powder (for example, tungsten, copper, or aluminum), a ceramic powder (for example, tungsten oxide or alumina), or a carbon allotrope powder (for example, graphite, graphene, carbon nanotubes, or diamond) with epoxy resin, or may be rubber. The backing material 420 may be mixed with a solvent, for example, water, so as to easily flow. Thus, the fluid including the backing material 420 may be contained in a chamber.

In operation S720, the foam-type body 410 is inserted into the fluid. The foam-type body 410 may be a porous foam type. The foam-type body 410 may include the plurality of pores 411 and the plurality of bridges 412 connected to one another between the pores 411. The bridges 412 are connected to one another and may include a highly heat-conductive material. For example, the bridges 412 may include metal. The metal may be a highly heat-conductive material, such as copper, aluminum, silver, nickel, iron, gold, or tungsten. The bridges 412 of the foam-type body 410 may be connected to one another without disconnections to serve as a passage via which heat generated in the ultrasound probe 300 is discharged to the outside. Thus, the heat generated in the ultrasound module 110 may be externally discharged via the foam-type body 410, for example, the bridges 412. The foam-type body 410 may include the pores 411. At least some of the pores 411 may overlap with each other. In other words, a pore 411 may be connected to at least one of neighboring pores 411. Accordingly, an external material may flow into the foam-type body 410 via the pores 411. A porosity of the foam-type body 410 may be about 50% or more. A diameter of each pore 411 may be, but is not limited to, about 1 mm to about 10 mm.

When the porous foam-type body 410 is inserted into the fluid, the fluid flows into the foam-type body 410 via the pores 411 of the foam-type body 410 to fill the pores 411. At least one of the fluid and the foam-type body 410 may be vibrated so that the backing material 420 is filled with the foam-type body 410. In other words, the chamber that contains the fluid and the foam-type body 410 is vibrated on a vibrator.

In operation S730, the foam-type body 410 filled with the backing material 420 is hardened to form the backing member 330. The backing member 330 manufactured in this way may be cut and joined with the ultrasound module 110 according to use purposes.

## Claims

1. An ultrasound probe (300), comprising:
an ultrasound module (110) comprising a transducer (220) configured to convert ultrasound waves into an electrical signal or vice versa; and
a backing member (330) including a porous foam-type body (410) and a backing material that absorbs the ultrasound waves generated by the ultrasound module,
**CHARACTERISED IN THAT**
the porous foam-type body (410) comprises a plurality of pores (411) and a plurality of bridges (412) which are connected to one another and located between the plurality of pores (411), and wherein the plurality of bridges (412) are formed of at least one material selected from a group of metals of copper, aluminum, silver, nickel, iron, gold, and tungsten, and
the backing member (330) is grounded.

2. The ultrasound probe of claim 1, wherein each pore (411) in the plurality of pores included in the foam-type body is connected to at least one of neighboring pores (411).

3. The ultrasound probe of any one of claims 1 to 2, wherein a diameter of at least one of the plurality of pores (411) included in the foam-type body is about 1 mm to about 10 mm.

4. The ultrasound probe of any one of claims 1 to 3, wherein a porosity of the foam-type body (410) is 50% or more.

5. The ultrasound probe of any one of claims 1 to 4, wherein the backing material comprises epoxy resin.

6. The ultrasound probe of any one of claims 1 to 5, wherein heat generated in the ultrasound module (110) is discharged to the outside via the backing member.

7. The ultrasound probe of any one of claim 1 to 6, wherein the transducer (220) comprises a piezoelectric element that converts the ultrasound waves into the electrical signal or vice versa, by vibrating.

8. The ultrasound probe of any one of claims 1 to 7, wherein the backing member (330) supports the ultrasound module (110).

## Patentansprüche

1. Ultraschallsonde (300), die Folgendes umfasst:
ein Ultraschallmodul (110) mit einem Wandler (220), der dazu konfiguriert ist, Ultraschallwellen in ein elektrisches Signal umzuwandeln oder umgekehrt; und
ein Trägerelement (330) mit einem porösen Schaumstoffkörper (410) und einem Trägermaterial, das die von dem Ultraschallmodul erzeugten Ultraschallwellen absorbiert,
**DADURCH GEKENNZEICHNET, DASS**
der poröse Schaumstoffkörper (410) eine Vielzahl von Poren (411) und eine Vielzahl von Stegen (412), die miteinander verbunden sind und zwischen der Vielzahl von Poren (411) angeordnet sind, umfasst, und wobei die Vielzahl von Stegen (412) aus wenigstens einem Material gebildet ist, das ausgewählt ist aus einer Gruppe von Metallen bestehend aus Kupfer, Aluminium, Silber, Nickel, Eisen, Gold und Wolfram, und
das Trägerelement (330) geerdet ist.

2. Ultraschallsonde nach Anspruch 1, wobei jede Pore (411) aus der Vielzahl von in dem Schaumstoffkörper enthaltenen Poren mit wenigstens einer von benachbarten Poren (411) verbunden ist.

3. Ultraschallsonde nach einem der Ansprüche 1 und 2, wobei ein Durchmesser von wenigstens einer aus der Vielzahl von in dem Schaumstoffkörper enthaltenen Poren (411) von etwa 1 mm bis etwa 10 mm beträgt.

4. Ultraschallsonde nach einem der Ansprüche 1 bis 3, wobei die Porosität des Schaumstoffkörpers (410) 50 % oder mehr beträgt.

5. Ultraschallsonde nach einem der Ansprüche 1 bis 4, wobei das Trägermaterial Epoxidharz umfasst.

6. Ultraschallsonde nach einem der Ansprüche 1 bis 5, wobei in dem Ultraschallmodul (110) erzeugte Wärmeenergie über das Trägerelement nach außen abgegeben wird.

7. Ultraschallsonde nach einem der Ansprüche 1 bis 6, wobei der Wandler (220) ein piezoelektrisches Element umfasst, das die Ultraschallwellen durch Vibration in das elektrische Signal umwandelt oder umgekehrt.

8. Ultraschallsonde nach einem der Ansprüche 1 bis 7, wobei dasTrägerelement (330) das Ultraschallmodul (110) trägt.

## Revendications

1. Sonde à ultrasons (300), comprenant :
un module à ultrasons (110) comprenant un transducteur (220) conçu pour convertir des ondes ultrasonores en un signal électrique ou vice versa, et
un élément de support (330) comportant un corps de type mousse poreuse (410) et un matériau de support qui absorbe les ondes ultrasonores générées par le module à ultrasons,
**CARACTÉRISÉE EN CE QUE**
le corps de type mousse poreuse (410) comprend une pluralité de pores (411) et une pluralité de ponts (412) reliés entre eux et situés parmi la pluralité de pores (411) ; ladite pluralité de ponts (412) se composant d'au moins un matériau issu du groupe constitué des métaux suivants : cuivre, aluminium, argent, nickel, fer, or et tungstène, et
l'élément de support (330) est mis à la masse.

2. Sonde à ultrasons selon la revendication 1, dans laquelle chaque pore (411) de la pluralité de pores présents dans le corps de type mousse est relié à au moins un pore (411) voisin.

3. Sonde à ultrasons selon l'une quelconque des revendications 1 et 2, dans laquelle le diamètre d'au moins un pore de la pluralité de pores (411) présents dans le corps de type mousse va d'environ 1 mm à environ 10 mm.

4. Sonde à ultrasons selon l'une quelconque des revendications 1 à 3, dans laquelle la porosité du corps de type mousse (410) est de 50 % ou plus.

5. Sonde à ultrasons selon l'une quelconque des revendications 1 à 4, dans laquelle le matériau de support comprend la résine époxyde.

6. Sonde à ultrasons selon l'une quelconque des revendications 1 à 5, dans laquelle la chaleur générée dans le module à ultrasons (110) est évacuée vers l'extérieur par l'élément de support.

7. Sonde à ultrasons selon l'une quelconque des revendications 1 à 6, dans laquelle le transducteur (220) comprend un élément piézoélectrique qui, en vibrant, convertit les ondes ultrasonores en ledit signal électrique ou vice versa.

8. Sonde à ultrasons selon l'une quelconque des revendications 1 à 7, dans laquelle l'élément de support (330) supporte le module à ultrasons (110).
